(19) Europäisches Patentamt
European Patent Office
Office européen des brevets

(11) Numéro de publication : **0 136 214**
**B1**

(12) **FASCICULE DE BREVET EUROPÉEN**

(45) Date de publication du fascicule du brevet : **30.12.86**

(51) Int. Cl.[4] : **C 07 C 69/96**, C 07 C 68/02, C 07 C 68/06

(21) Numéro de dépôt : **84401672.5**

(22) Date de dépôt : **14.08.84**

# (54) Procédé de préparation de chloroformiates alpha-chlorés.

(30) Priorité : **26.08.83 FR 8313795**

(43) Date de publication de la demande : **03.04.85 Bulletin 85/14**

(45) Mention de la délivrance du brevet : **30.12.86 Bulletin 86/52**

(84) Etats contractants désignés : **AT CH DE FR GB LI SE**

(56) Documents cités : **EP-A- 0 034 536**
**FR-A- 2 482 587**

(73) Titulaire : **SOCIETE NATIONALE DES POUDRES ET EXPLOSIFS**
**12, quai Henri IV**
**F-75181 Paris Cedex 04 (FR)**

(72) Inventeur : **Malfroot, Thierry**
**1 bis Route de Tigery**
**F-91100 Saint Germain Les Corbeil (FR)**
Inventeur : **Senet, Jean-Pierre**
**79 rue de la Gare Herbeauvilliers-Buthiers**
**F-77760 La Chapelle La Reine (FR)**

**EP 0 136 214 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention concerne la fabrication de chloroformiates α-chlorés.

Pendant longtemps ces composés, de formule générale :

$$R-\left(\begin{array}{l}CH-O-COCl\\ \ | \\ Cl\end{array}\right)_n$$

n'ont été préparés que par chloration photochimique des chloroformiates $RCH_2OCOCl$, avec formation simultanée de nombreux dérivés secondaires plus ou moins faciles à séparer du produit cherché. Récemment, un nouveau procédé, qui permet d'obtenir des dérivés α-chlorés sensiblement purs, a été décrit dans des demandes de brevet français de la demanderesse, publiées sous les n° 2.482.587 et 2.516.075.

Ce dernier procédé prévoit notamment l'action du phosgène sur des aldéhydes de structures très variées en présence d'un catalyseur capable de générer dans le milieu réactionnel une paire d'ions dont l'un est un halogénure et l'autre un cation suffisamment séparé dudit anion pour impartir à ce dernier un pouvoir nucléophile lui permettant d'attaquer la ou les fonctions aldéhydes. Ce procédé entraîne la nécessité de manipuler des quantités importantes de phosgène, impliquant des mesures de sécurité très spécifiques tant en ce qui concerne le transport et le stockage du réactif que sa mise en réaction. Ce réactif, très toxique a en effet une tension de vapeur particulièrement élevée dès la température ambiante.

Il était donc souhaitable de disposer d'un autre procédé général de préparation des chloroformiates α-chlorés, plus facile à mettre en œuvre en particulier en ce qui concerne les conditions de travail et de sécurité.

Le procédé selon la présente invention est applicable à la préparation d'un grand nombre de chloroformiates α-chlorés et sans qu'il soit nécessaire d'opérer avec les conditions strictes de sécurité imposées par l'emploi de phosgène.

Le procédé de l'invention concerne la préparation de chloroformiates α-chlorés de formule :

$$R-\left(\begin{array}{l}CH-O-COCl\\ \ | \\ Cl\end{array}\right)_n$$

dans laquelle R représente l'atome d'hydrogène, un radical hydrocarboné aliphatique, en particulier en C1 à C12, aromatique ou hétérocyclique substitué ou non et n un nombre entier, est caractérisé en ce qu'on fait réagir sur un aldéhyde de formule $R(CHO)_n$, un dérivé trichlorométhylé, choisi parmi le chloroformiate de trichlorométhyle $ClCOOCCl_3$ et le carbonate de di(trichlorométhyle) $Cl_3COCOOCCl_3$, en présence d'un composé capable de libérer dans le milieu, directement ou non, une paire d'ions, dont l'anion est un halogénure et dont le pouvoir attracteur du cation sur l'ion de charge opposée est suffisamment faible pour que l'anion attaque la fonction aldéhyde, le cation étant en outre non susceptible de réagir sur les fonctions chloroformiates du milieu.

La réaction peut avoir lieu avec ou sans tiers solvant.

Le dérivé trichlorométhylé utilisé a une tension de vapeur nettement inférieure à celle du phosgène et les risques encourus lors de la manipulation sont bien moindres. Sa préparation peut être effectuée en utilisant par exemple le procédé décrit dans Organic Synthesis *59* p. 195-201 Ed. John Wiley and Sons, qui consiste à réaliser une chloration photochimique du chloroformiate de méthyle ou du carbonate de diméthyle.

Le procédé selon la présente invention peut être appliqué à des composés porteurs d'une seule fonction aldéhyde et il convient aussi pour des composés porteurs de plusieurs fonctions.

Comme aldéhyde, on peut citer le formaldéhyde qui réagit quantitativement si l'on prend les précautions nécessaires pour éviter sa polymérisation mais aussi les acétaldéhyde, valéraldéhyde, chloral, acroléine et cyclohexylcarboxaldéhyde ou encore le benzaldéhyde, le chloro-2-benzaldéhyde ou l'aldéhyde téraphtalique. Cette liste n'est pas limitative, d'autres aldéhydes plus lourds ou plus complexes pouvant aussi être utilisés comme matière première.

Comme composé susceptible de libérer dans le milieu la paire d'ions convenables, on peut citer la pyridine et les alkylpyridines, les amides disubstituées à l'azote, les urées, les thiourées tétrasubstituées et avantageusement les tétrabutylurée et tétraméthylurée, les phosphoramides substitués à l'azote dont l'hexaméthylphosphotriamide, et leurs produits de réaction avec des agents chlorurants dont le dérivé trichlorométhylé tel que précédemment défini.

On peut encore citer les halogénures d'ammonium quaternaires :

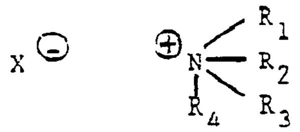

où X représente un halogène, les radicaux $R_1$, $R_2$, $R_3$, $R_4$ sont des groupes hydrocarbonés dont la somme des atomes de carbone est supérieure ou égale à 16 et de préférence tels que chaque groupe a au moins 4 atomes de carbone, afin que l'azote porteur de la charge positive soit suffisamment masqué par des substituants volumineux à son environnement immédiat, comme dans le chlorure de tributylbenzylammonium.

On peut également utiliser un halogénure métallique, de préférence d'un métal alcalin ou alcalino-terreux, dont le cation est sequestré par un éther couronne ou un cryptant, tel que l'éther couronne 18/6 ou le diaza-1,10 hexaoxa-4,7,13,16,21,24-bicyclo(8,8,8)hexacozane et le chlorure de potassium.

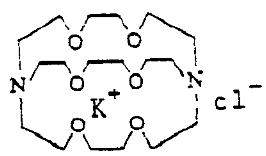

Dans ce qui précède par halogénure, on entend un chlorure, bromure ou iodure, le chlorure étant préféré.

Dans la mise en œuvre du procédé de l'invention, les quantités de ce composé introduit dans le milieu seront adaptées à la nature de l'aldéhyde utilisé, et également à la nature du composé.

On utilisera en général de 0,5 à 10 % en mole de la substance par rapport à l'aldéhyde et de préférence de 2 à 7 %. Mais dans des cas particuliers, on pourra ajouter jusqu'à 50 % de ce composé sans inconvénients majeurs.

La réaction selon l'invention peut être effectuée sans solvant, notamment lorsque les réactifs sont liquides à la température choisie, ou dans un solvant inerte, c'est-à-dire aprotique et peu ou pas polaire, comme des hydrocarbures aliphatiques, chlorés ou non (tétrachlorure de carbone, chloroforme, chlorure de méthylène ou hexane) ou des hydrocarbures aromatiques comme le toluène et le chlorobenzène. Il importe de n'utiliser que des solvants anhydres et exempts d'acide chlorhydrique dissous sous peine de voir diminuer très sensiblement le rendement du procédé. Le milieu réactionnel sera de préférence maintenu à une température comprise entre —10 °C et 100 °C et plus particulièrement entre 0 °C et 70 °C, cette température étant notamment fonction de la fragilité du produit final.

Le mode opératoire préconisé pour effectuer la synthèse selon le procédé de la présente invention consiste le plus souvent à mélanger dans un réacteur à l'abri de l'humidité l'aldéhyde et le composé additionnel libérant l'anion halogénure, en présence ou non d'un tiers solvant, à refroidir si nécessaire puis à introduire ensuite dans le milieu le dérivé trichlorométhyle et enfin à chauffer le mélange si nécessaire pour le maintenir à la température de réaction convenable.

L'invention est illustrée par les exemples qui suivent, mais elle ne saurait s'y limiter et notamment le procédé selon l'invention peut être appliqué à des quantités de réactifs notablement plus importantes. Le procédé de l'invention permet l'obtention aisée de chloroformiates α-chlorés qui sont notamment utilisés dans la fabrication de certaines pénicillines et céphalosporines dont il est utile de protéger les fonctions acides carboxyliques pour améliorer les propriétés thérapeutiques de la structure de base. Les chloroformiates α-chlorés sont également utilisables pour la N-déalkylation sélective des amines tertiaires dans la série des morphinanes, comme décrit par exemple dans le Journal of Organic Chem. 1984-49 p. 2081-2082.

## Exemple 1

Chloroformiate de chloro-1 éthyle.

La réaction mise en jeu est la suivante :

$$_2CH_3CHO + Cl_3COCOCl \longrightarrow {}_2CH_3CHCLOCOCl$$

Dans un réacteur de 50 ml muni d'un agitateur, d'un réfrigérant à reflux équipé d'une garde à chlorure de calcium, on introduit 3,52 g (0,08 mole) d'acétaldéhyde et 1,6 g (0,004 mole) de chlorure de chlorotétrabutylimonium

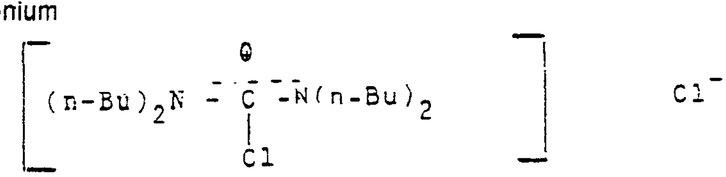

qui peut être préparé par chauffage de tétrabutylurée et d'un agent chlorurant tel que par exemple $POCl_3$, $COCl_2$, $Cl_3COCOCl$ et $Cl_3COCOOCCl_3$.

On introduit goutte à goutte, sous agitation, en 20 minutes environ, 9,5 g de $Cl_3COCOCl$ (0,048 mole) en maintenant la température entre 20 et 30 °C. L'agitation est prolongée 1/2 heure après la fin de l'addition.

On analyse alors le milieu réactionnel par spectrométrie infrarouge et de résonance magnétique nucléaire sur un prélèvement d'une partie aliquote. On constate ainsi que tout l'acétaldéhyde a réagi (disparition en RMN du quadruplet caractéristique à 9,7 ppm) et s'est transformé en chloroformiate de chloro-1 éthyle (en RMN doublet à 1,85 ppm correspondant au proton du $CH_3$ et quadruplet à 6,44 ppm pour le proton du CH) ; la fréquence caractéristique du carbonyle du carbonate est de 1 780 $cm^{-1}$.

La réaction étant complète, on a séparé les insolubles et isolé le produit final par distillation. Sa température d'ébullition est de 68 °C sous 18 950 Pa (150 mm Hg).

Exemple 2

Chloroformiate de chloro-1 éthyle

On opère comme dans l'exemple 1 mais la substance additionnelle est cette fois de la pyridine.

On fait ainsi réagir 3,1 g (0,07 mole) d'acétaldéhyde, 0,3 g (0,004 mole) de pyridine et 8,5 g (0,043 mole) de chloroformiate de trichlorométhyle. La température est maintenue 2 heures entre 20 et 25 °C après la fin d'addition du chloroformiate. L'analyse RMN du milieu réactionnel indique à ce moment la formation de produit final avec un rendement de 96,3 %.

Exemple 3

Chloroformiate de chloro-1 éthyle

On opère comme à l'exemple précédent mais en utilisant 1,1 g (0,003 5 mole) de chlorure de benzyl tri n-butylammonium anhydre, au lieu de pyridine.

Le rendement de la préparation, déterminé par RMN, est de 81,3 %.

Exemple 4

Chloroformiate de chloro-1 éthyle

On opère comme dans l'exemple 3, mais sur une masse réactionnelle supérieure.

On fait réagir 21,5 g (0,48 mole) d'acétaldéhyde, 7,6 g (0,024 mole) de chlorure de benzyle tri(n-butyl)ammonium et 53 g (0,27 mole) de chloroformiate de trichlorométhyle. Après la fin de la réaction, le mélange réactionnel est distillé sous pression réduite (2 660 Pa) pour donner 60,6 g d'une fraction ayant distillé entre 20 et 40 °C. Une deuxième distillation de cette fraction sous pression atmosphérique donne 52,4 g de chloroformiate de chloro-1 éthyle, de point d'ébullition 117 °C et indice $n_D^{20} = 1{,}422\,0$. Le rendement global est de 77 %.

Exemple 5

Chloroformiate de chloro-1 éthyle en présence d'éther couronne et chlorure de potassium.

On opère comme dans l'exemple 2 mais en utilisant à la place de la pyridine un mélange de 0,5 g (0,007 mole) de chlorure de potassium et de 1 g (0,000 4 mole) d'éther couronne 18-6 commercialisé par MERCK-Darmstadt (RFA).

Le rendement de la réaction, déterminé par RMN, est de 52,4 %.

Exemple 6

Chloroformiate d'α-chlorobenzyle

Dans un appareillage analogue à celui de l'exemple 1, on introduit 7,5 g (0,07 mole) de benzaldéhyde et 0,3 g (0,004 mole) de pyridine. On porte le mélange à 45 °C et introduit goutte à goutte, en 10 minutes 8,5 g (0,043 mole) de chloroformiate de trichlorométhyle. Après 2 heures d'agitation à 45 °C, on constate par analyse RMN que tout l'aldéhyde s'est transformé en chloroformiate α-chloré de formule.

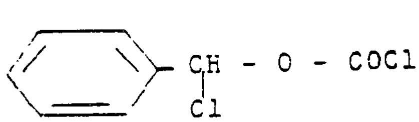

Exemple 7

Chloroformiate de chlorométhyle

La réaction mise en jeu est la suivante :

$$2HCHO + Cl_3COCOCl \longrightarrow 2\,Cl{-}CH_2OCOCl$$

Les conditions opératoires sont spécifiques à la préparation de ce composé étant donné la réactivité particulière du formaldéhyde et notamment sa facilité de polymérisation.

Dans un appareillage identique à celui de l'exemple 1 on introduit d'abord 7 g (0,035 mole) de chloroformiate de trichlorométhyle et 1,4 g (0,004 mole) de chlorure de benzyl tri(n-butyl)ammonium anhydre, tout en refroidissant le milieu à 0 °C, puis 2,5 g de formaldéhyde, obtenu par chauffage de 2,5 g de paraformaldéhyde préalablement séché sous vide de 13 Pa en présence de $P_2O_5$. Les vapeurs de formaldéhyde sont introduites dans le milieu réactionnel sous la surface, par l'intermédiaire d'un tube plongeant.

Après une heure d'agitation à 0 °C, on constate par analyse RMN, que la réaction est totale (singulet à 5,7 ppm).

**Revendications**

1. Procédé de préparation de chloroformiates α-chlorés de formule :

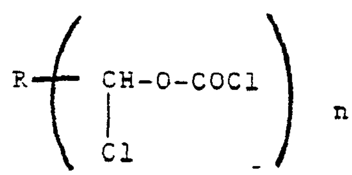

dans laquelle R représente l'atome d'hydrogène, un radical hydrocarboné aliphatique, aromatique ou un radical hétérocyclique, substitué ou non et n un nombre entier caractérisé en ce que l'on fait réagir sur l'aldéhyde de formule $R(CHO)_n$ un dérivé trichlorométhylé, choisi parmi le chloroformiate de trichlorométhyle $ClCOOCCl_3$ et le carbonate de di-trichlorométhyle $Cl_3COCOOCCl_3$, en présence d'un composé libérant, directement ou non, dans le milieu une paire d'ions, dont l'anion est un halogénure et dont le pouvoir attracteur du cation sur l'ion de charge opposé est suffisamment faible pour que l'anion attaque la fonction aldéhyde, le cation n'étant en outre pas susceptible de réagir sur les fonctions chloroformiates du milieu.

2. Procédé selon la revendication 1, caractérisé en ce que le composé est choisi dans le groupe constitué par la pyridine et les alkylpyridines, les amides N-N disubstituées, les urées et thiourées tétrasubstituées, les phosphoramides substituées à l'azote ainsi que leurs produits de réaction avec des agents chlorurants dont le dérivé trichlorométhylé lui-même, les halogénures d'ammonium quaternaire volumineux ou les halogénures de métaux alcalins ou alcalino-terreux dont le cation est séquestré par un éther couronne et un cryptant.

3. Procédé selon l'une quelconque des revendications 1 à 2, caractérisé en ce que le composé est utilisé en proportion comprise entre 0,5 et 50 % en mole de l'aldéhyde, de préférence entre 2 à 7 %.

4. Procédé selon l'une quelconque des revendications 1 à 3, caractérisé en ce que la réaction est effectuée sans solvant, en milieu anhydre et en l'absence d'acide chlorhydrique.

5. Procédé selon l'une quelconque des revendications 1 à 3, caractérisé en ce que la réaction est effectuée dans un solvant aprotique peu polaire ou non polaire, en l'absence de traces d'eau et d'acide chlorhydrique.

6. Procédé selon l'une quelconque des revendications 1 à 5, caractérisé en ce qu'il est conduit à une température de — 10 °C à 100 °C, en particulier entre 0 et 70 °C.

**Claims**

1. Process for preparing α-chlorinated chloroformates of formula :

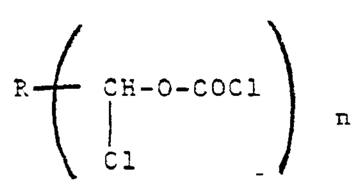

in which R denotes a hydrogen atom, an aliphatic or aromatic hydrocarbon radical or a heterocyclic radical, the radical being substituted or unsubstituted, and n an integer, characterized in that an aldehyde of formula $R(CHO)_n$ is reacted with a trichloromethyl derivative, chosen from trichloromethyl chloroformate $ClCOOCCl_3$ and bis(trichloromethyl) carbonate $Cl_3COCOOCCl_3$, in the presence of a compound which releases into the medium, directly or indirectly, an ion pair in which the anion is a halide and in which the attracting power of the cation on the ion of opposite charge is sufficiently low for the anion to attack the aldehyde group, the cation, in addition, not being capable of reacting with the chloroformate groups of the medium.

2. Process according to Claim 1, characterized in that the compound is chosen from the group consisting of pyridine and alkylpyridines, N,N-disubstituted amides, tetrasubstituted ureas and thioureas, phosphoramides substituted on the nitrogen and also their products of reaction with chlorinating agents including the trichloromethyl derivative itself, bulky quaternary ammonium halides or alkali metal or alkaline earth metal halides, in which the cation is sequestered by a crown ether and a cryptand.

3. Process according to either of Claims 1 and 2, characterized in that the compound is used in a proportion of between 0.5 and 50 mol % of the aldehyde, and preferably between 2 % and 7 %.

4. Process according to any one of Claims 1 to 3, characterized in that the reaction is performed without a solvent, in anhydrous medium and in the absence of hydrochloric acid.

5. Process according to any one of Claims 1 to 3, characterized in that the reaction is performed in an aprotic solvent which is only slightly polar or nonpolar, in the absence of traces of water and hydrochloric acid.

6. Process according to any one of Claims 1 to 5, characterized in that it is performed at a temperature of —10 °C to 100 °C, especially between 0 °C and 70 °C.

**Patentansprüche**

1. Verfahren zur Herstellung von α-chlorierten Chlorameisensäureestern der Formel

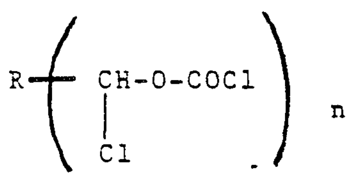

in der R ein Wasserstoffatom, einen aliphatischen oder aromatischen Kohlenwasserstoffrest oder einen heterocyclischen Rest, ggf. substituiert, und n eine ganze Zahl bedeutet, gekennzeichnet durch Umsetzung eines Aldehyds der Formel $R(CHO)_n$ und eines Trichlormethylderivats, ausgewählt unter Chlorameisensäure-trichlormethylester $ClCOOCCl_3$ und Kohlensäuredi-trichlormethylester $Cl_3CO$-$COOCCl_3$, in Gegenwart einer Verbindung, die im Medium direkt oder indirekt ein Ionenpaar freisetzt, dessen Anion ein Halogenid ist und dessen Kation eine genügend schwache Anziehung auf auf das Ion der entgegengesetzten Ladun hat, daß das Anion die Aldehydgruppe angreift, wobei das Kation aber nicht mit den Chlorameisensäureester-Gruppen des Mediums reagieren kann.

2. Verfahren nach Anspruch 1, dadurch gekennzeichnet, daß die Verbindung unter Pyridin und Alkylpyridinen, N,N-substituierten Amiden, tetrasubstituierten Harnstoffen und Thioharnstoffen, N-substituierten Phosphoramiden sowie ihren Reaktionsprodukten mit chlorierenden Mitteln, wie ihrem Trichlormethylderivat, voluminösen quartären Ammoniumhalogeniden oder Alkali- oder Erdalkalimetallhalogeniden, deren Kation von einem Kronenether oder Kryptanden gebunden ist, ausgewählt wird.

3. Verfahren nach Anspruch 1 oder 2, dadurch gekennzeichnet, daß die Verbindung in einer Menge von 0,5 bis 50 und vorzugsweise von 2 bis 7 mol-%, bezogen auf den Aldehyd, verwendet wird.

4. Verfahren nach einem der Ansprüche 1 bis 3, dadurch gekennzeichnet, daß die Reaktion ohne Lösungsmittel, in wasserfreiem Medium und in Abwesenheit von Chlorwasserstoff durchgeführt wird.

5. Verfahren nach einem der Ansprüche 1 bis 3, dadurch gekennzeichnet, daß die Reaktion in einem schwach-oder nicht-polaren Lösungsmittel in Abwesenheit von Spuren von Wasser oder Chlorwasserstoff durchgeführt wird.

6. Verfahren nach einem der Ansprüche 1 bis 5, gekennzeichnet, durch die Anwendung einer Temperatur von —10 bis 100 °C und vorzugsweise von 0 bis 70 °C.